(19) Europäisches Patentamt
European Patent Office
Office européen des brevets

(11) Veröffentlichungsnummer: **0 249 858**
**B1**

(12) **EUROPÄISCHE PATENTSCHRIFT**

(45) Veröffentlichungstag der Patentschrift: **05.12.90**

(51) Int. Cl.[5]: **C 07 D 215/48,** A 01 N 43/42

(21) Anmeldenummer: **87108325.9**

(22) Anmeldetag: **11.06.87**

# (54) Fluoralkylsubstituierte Chinolinderivate. ihre Herstellung sowie Verwendung zur Bekämpfung unerwünschten Pflanzenwachstums.

(30) Priorität: **14.06.86 DE 3620064**

(43) Veröffentlichungstag der Anmeldung:
**23.12.87 Patentblatt 87/52**

(45) Bekanntmachung des Hinweises auf die Patenterteilung:
**05.12.90 Patentblatt 90/49**

(84) Benannte Vertragsstaaten:
**DE FR GB IT**

(56) Entgegenhaltungen:
**EP-A-0 104 389**

(73) Patentinhaber: **BASF Aktiengesellschaft**
**Carl-Bosch-Strasse 38**
**D-6700 Ludwigshafen (DE)**

(72) Erfinder: **Hagen, Helmut, Dr.**
**Max-Slevogt-Strasse 17 e**
**D-6710 Frankenthal (DE)**
Erfinder: **Eichenauer, Ulrich, Dr.**
**Paul-Ehrlich-Strasse 25 a**
**D-6000 Frankfurt 70 (DE)**
Erfinder: **Kohler, Rolf-Dieter, Dr.**
**Amselweg 3**
**D-6803 Edingen-Neckarhausen (DE)**
Erfinder: **Plath, Peter, Dr.**
**Hans-Balcke-Strasse 13**
**D-6710 Frankenthal (DE)**
Erfinder: **Liese-Sauer, Thomas, Dr.**
**Sigmund-Hirsch-Platz 10**
**D-6940 Weinheim (DE)**
Erfinder: **Eicken, Karl, Dr.**
**Am Huettenwingert 12**
**D-6706 Wachenheim (DE)**
Erfinder: **Wuerzer, Bruno, Dr.**
**Ruedigerstrasse 13**
**D-6701 Otterstadt (DE)**
Erfinder: **Meyer, Norbert, Dr.**
**Dossenheimer Weg 22**
**D-6802 Ladenburg (DE)**

**EP 0 249 858 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue fluoralkylsubstituierte Chinolinderivate, Verfahren zu ihrer Herstellung, Herbizide, die diese Verbindungen als Wirkstoffe enthalten, sowie ein Verfahren zur Bekämpfung unerwünschten Pflanzenwuchses.

Herbizid wirksame Chinolin-8-carbonsäuren, beispielsweise 3-Halogenalkyl-7-halogen(5,7-dihalogen)chinolin-8-carbonsäuren, sind aus EP—A—O 104 389 bekannt. Als Vertreter dieser Verbindungsklasse sind aber nur 3-Brommethyl-7-chlor(5,7-dichlor)chinolin-8-carbonsäure und deren Alkylester beschrieben.

Es wurde nun gefunden, daß fluoralkylsubstituierte Chinolinderivate der Formel I

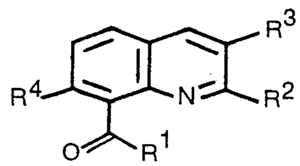

(I),

in der

$R^1$ für Chlor, Fluor, Hydroxy, $O^{\ominus}Met^{\oplus}$, $C_1$—$C_4$-Alkoxy oder einen Rest der Formel

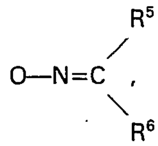

,

$R^2$, $R^3$ unabhängig voneinander für Wasserstoff, $C_1$—$C_3$-Alkyl, welches an einem der Kohlenstoffatome 2 oder Fluoratome tragen kann;

$R^4$ für Fluor, Chlor oder Brom stehen, wobei

$Met^{\oplus}$ ein Alkalimetallion und

$R^5$, $R^6$ unabhängig voneinander $C_1$—$C_3$-Alkyl bedeuten,

mit der Maßgabe, daß mindestens einer der Reste $R^2$ und $R^3$ $C_1$—$C_3$-Fluoralkyl bedeutet, herbizid wirksamer und gegenüber wichtigen Kulturpflanzen selektiver sind als die aus EP—A—O 104 389 bekannten 3-Brommethyl-chinolinderivate.

In Formel I bedeutet $R^1$ außer Chlor, Fluor, Hydroxy beispielsweise $O^{\ominus}Na^{\oplus}$, $O^{\ominus}K^{\oplus}$, Methoxy, Ethoxy, n-Propoxy, iso-Propoxy, N-Butoxy oder einen Rest der Formel

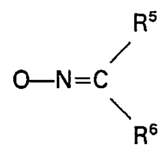

,

worin $R^5$ und $R^6$ unabhängig voneinander für Methyl, Ethyl oder n-Propyl stehen. Bevorzugte Reste $R^1$ sind Hydroxy oder der Rest der Formel

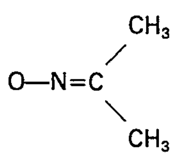

$R^2$ und $R^3$ in Formel I bedeuten beispielsweise Wasserstoff, Methyl, Ethyl n-Propyl, iso-Propyl oder $C_1$—$C_3$-Fluoralkyl, wie Difluormethyl, Trifluormethyl, 1,1-Difluorethyl, 2,2-Difluorethyl, 2,2,2-Trifluorethyl oder 3,3,3-Trifluor-n-propyl, wobei mindestens einer der Reste $R^2$ und $R^3$ Fluoralkyl bedeutet. Bevorzugter Fluoralkylrest ist Trifluormethyl.

$R^4$ in Formel I bedeutet Fluor, Chlor oder Brom, vorzugsweise Chlor.

Man erhält die Chinolinderivate der Formel I in denen $R^1$ Hydroxy bedeutet, durch Umsetzung von Verbindungen der Formel II

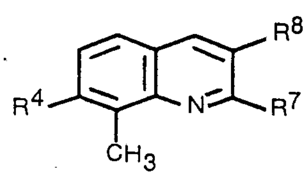

(II),

in der

$R^4$ die oben angegebenen Bedeutungen hat, und mindestens einer der Substituenten

$R^7$ oder $R^8$ für Formyl, Acetyl, Carboxyl, Carboxymethyl, Carboxyethyl oder Formylmethyl steht und gegebenenfalls der andere Substituent Wasserstoff oder $C_1$—$C_3$-Alkyl bedeutet,

mit Schwefeltetrafluorid, und anschließende Oxidation der 8-Methylgruppe zu Carboxyl.

Die Reaktion mit Schwefeltetrafluorid, durch welche die Struktur C—OH in C—F und C=O in $CF_2$ überführt wird, wird in Gegenwart eines inerten Verdünnungsmittels, beispielsweise eines halogenierten Kohlenwasserstoffs, wie Methylenchlorid, Chloroform, Tetrachlorkohlenstoff, Dichlorethan, Trichlorethan, Hexachlorethan, Trifluortrichlorethan, oder eines Aromaten, wie Benzol, Chlorbenzol, Toluol, in Gegenwart von 0 bis 100 Äquivalenten Fluorwasserstoffsäure, mit 1 bis 20 Äquivalenten Schwefeltetrafluorid unter erhöhtem Druck und erhöhter Temperatur durchgeführt. Bevorzugt sind chlorierte Kohlenwasserstoffe und 10—50 Äquivalente Fluorwasserstoffsaüre als Reaktionsmedium, 80—160°C als Reaktionstemperatur. Die Oxidation von Methyl zu Carboxyl kann ein- oder mehrstufig erfolgen. Bevorzugt ist die Bromierung der Methylgruppe und anschließende Oxidation.

Dazu wird die Verbindung mit der Methylgruppe in 8-Stellung mit einem Halogenierungsmittel, wie Brom, Chlor, N-Bromsuccinimid, N-Chlorsuccinimid, N-Chloracetamid, N-Bromacetamid, N-Chlorphthalimid oder N-Bromphthalimid, in Gegenwart eines inerten Verdünnungsmittels bei erhöhter Temperatur umgesetzt, wobei die typischen Bedingungen einer Radikalreaktion (Anwesenheit von Radikalstarter, Belichtung) vorteilhaft, aber nicht zwingend notwendig sind.

Inerte Verdünnungsmittel können perhalogenierte Kohlenwasserstoffe, wie Tetrachlormethan, Hexachlorethan, Trifluortrichlorethan, oder chlorierte Aromaten, wie Chlorbenzol oder Dichlorbenzol, sein. Die Reaktionstemperatur kann je nach Verdünnungsmittel zwischen 60 und 180°C gewählt werden; als Radikalstarter sind Azoverbindungen, wie Azobis(isobuttersäurenitril), oder Peroxide, wie Dibenzoylperoxid, geeignet. Zur Belichtung genügt diffuses Sonnenlicht, es kann jedoch durch Glühlampen verstärkt werden.

Bevorzugte Reaktionsbedingungen sind: Chlorbenzol als Reaktionsmedium, Temperaturen zwischen 80 und 120°C, besonders bevorzugt zwischen 90 und 110°C, Azobis(isobuttersäurenitril) als Radikalstarter.

Die Oxidation von 8-Brommethylchinolinen zu Chinolin-8-carbonsäuren erfolgt in an sich üblicher Weise durch Umsetzung mit Salpetersäure in Schwefelsäure oder in einer Carbonsäure wie Propionsäure, zweckmäßigerweise bei 100 bis 140°C.

Analog erhält man die Chinolinderivate der Formel I auch durch Umsetzung einer entsprechenden Chinolin-8-carbonsäure der Formel IIa mit Schwefeltetrafluorid und gegebenenfalls anschließender Hydrolyse des gebildeten Chinolin-8-carbonsäurefluorids in an sich bekannter Weise.

2-Trifluormethylchinolin-8-carbonsäuren lassen sich durch Umsetzung von 2-Trifluormethyl-3,1-benzoxazin-4-onen mit Enaminen gemäß dem in Angew.

Chem. *85*, 505 (1973) beschriebenen Verfahren erhalten. Dazu werden substituierte 2-Trifluormethyl-3,1-benzoxazinone der Formel III, erhalten aus den entsprechenden Anthranilsäuren durch Cyclisierung mit Trifluoressigsäureanhydrid,

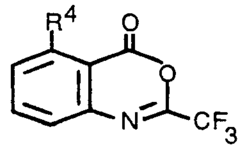

(III),

wobei $R^4$ die obengenannten Bedeutungen hat, mit Enaminen der Formel (IV)

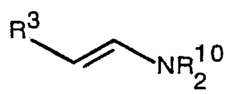

(IV),

in der $R^3$ $C_1$—$C_3$-Alkyl und $NR_2^{10}$ Di($C_1$—$C_3$-alkyl)-amino bedeutet, in polaren, aprotischen Lösungsmitteln, wie Acetonitril, Dimethylformamid, N-Methylpyrrolidon oder Tetrahydrofuran, bei Temperaturen zwischen −10°C und +50°C umgesetzt. Bevorzugt sind Acetonitril als Lösungsmittel und Temperaturen zwischen 0°C und 30°C.

Die folgenden Beispiele sollen die Erfindung erläutern, ohne sie einzuschränken.

## Beispiel 1

3-Trifluormethyl-7-chlor-chinolin-8-carbonsäure (Nr. 1 in Tab. 1)

1a) Zu einer Lösung von 58 g (0.23 mol) 7-Chlorchinolin-3,8-dicarbonsäure in 200 ml Methylenchlorid gibt man in einem Hasteloy-C-Rührautoklaven (1.2 l Fassungsvermögen) 120 g Fluorwasserstoffsäure und 140 g Schwefeltetrafluorid und läßt 10 Stunden bei 120°C rühren. Nach dem Abkühlen werden die flüchtigen Bestandteile am Rotationsdampfer abgezogen. Der Rückstand wird auf Eis gegossen und mit Methylenchlorid extrahiert. Ein unlöslicher Feststoff wird durch Filtration abgetrennt und verworfen. Nach Trocknung der Methylenchloridphase über Magnesiumsulfat wird das Lösungsmittel abgedampft und der erhaltene Rückstand wird aus n-Hexan umkristallisiert. Man erhält 27 g (42%) 3-Trifluormethyl-7-chlorchinolin-8-carbonsäurefluorid vom Schmp. 98—99°C.

1b) 10 g (36 mmol) des unter a) erhaltenen Säurefluorids werden mit 70 ml 5%iger Natronlauge zusammengegeben und 5 Min. bei 100°C gerührt. Nach Filtration von ungelösten Bestandteilen wird das Filtrat mit konz. Salzsäure angesäuert (pH 3). Der ausfallende Feststoff wird durch Absaugen isoliert und mit Wasser gewaschen. Nach dem Trocknen bei 50°C unter vermindertem Druck erhält man 7,4 g (75%) 3-Trifluormethyl-7-chlorchinolin-8-carbonsäure vom Schmp. 250°C.

Beispiel 2

Acetonoximester von 3-Trifluormethyl-7-chlor-chinolin-8-carbonsäure (Nr. 2 in Tab. 1)

Zu einer Lösung von 16.7 g (50 mmol) des unter 1a) erhaltenen Säurefluorids in 100 ml 1,1,1-Trichlorethan werden 3,65 g (50 mmol) Acetonoxim und 7.9 g (0,1 mol) Pyridin gegeben und anschließend 3 Std. bei 70°C gerührt. Nach dem Abkülen wird mit 5%iger Salzsäure extrahiert, mit Natriumhydrogencarbonat-Lösung entsäuert, über Magnesiumsulfat getrocknet und eingengt. Der verbleibende ölige Rückstand wird nach Anrühren mit Diethylether kristallin; Schmp.: 105—107°C; Ausbeute: 8.5 g (51%).

Beispiel 3

2-Trifluormethyl-3-ethyl-7-chlor-chinolin-8-carbonsäure (Nr. 3 in Tab. 1)

a) 5-Chlor-2-trifluormethyl-3,1-benzoxazin-4-on 6-Chloranthranilsäure wird mit Trifluoressigsäureanhydrid acyliert und mit siedendem Acetanhydrid cyclisiert.

Schmp.: 91—93%; Ausbeute 86%.

b) Zu einer Lösung von 10.0 g (40 mmol) von 5-Chlor-2-trifluormethyl-3,1-benzoxazin-4-on in 50 ml trockenem Acetonitril wird eine Lösung von 5,1 g (40 mmol) 1-Diethylaminobut-1-en in 10 ml Acetonitril bei 0°C getropft. Nach 16 Std. Rühren bei Raumtemperatur wird der ausgefallene Feststoff durch Absaugen isoliert, mit Diethylether gewaschen und in 30 ml Wasser suspendiert. Nach Zugabe von 12 ml 2 N NaOH wird filtriert, das Filtrat wird mit 2 N Salzsäure auf pH 3 angesäuert. Der ausgefallene Feststoff wird isoliert, mit Wasser gewaschen und bei 60°C unter vermindertem Druck getrocknet.

Ausbeute: 4.6 g (38%); 178—180°C.

Beispiel 4

2-Trifluormethyl-3-methyl-7-chlor-chinolin-8-carbonsäure (Nr. 4 in Tab. 1)

Analog Beispiel 3 wird aus 5-Chlor-2-trifluormethyl-3,1-benzoxazin-4-on und 1-Diethylamino-prop-1-en 2-Trifluormethyl-3-methyl-7-chlor-chinolin-8-carbonsäure hergestellt; Schmp.: 186—188°C; Ausbeute: 41%.

Beispiel 5

2,3-Bis(trifluormethyl-7-chlor-chinolin-8-carbonsäure (Nr. 5in Tab. 1)

a) 2,3-Bis(trifluormethyl-7-chlor-8-methylchinolin

In einem Hasteloy-C Rührautoklaven mit 0.5 l Fassungsvermögen werden 50 g 7-Chlor-8-methylchinolin-2,3-dicarbonsäure, 120 ml Methylenchlorid, 45 g Fluorwasserstoffsäure und 190 g Schwefeltetrafluorid für 10 Std. auf 120°C erhitzt. Nach abkühlen wird in Eiswasser gegeben, mit Methylenchlorid ausgeschüttelt und das Lösungsmittel nach Trocknen am Rotationsverdampfer entfernt. Zur Reinigung wird das Rohprodukt mit Pentan über Kieselgel adsorptiv filtriert; man isoliert 34.8 g (58%) 2,3-Bis(trifluormethyl)-7-chlor-8-methylchinolin vom Schmp. 70—73°C.

b) 2,3-Bis(trifluormethyl)-8-brommethyl-7-chlorchinolin

3.1 g der Verbindung aus a) werden in 30 ml Chlorbenzol mit 4.45 g N-Bromsuccinimid und 0.2 g Azobis(isobuttersäurenitril) 3 Std. unter Rückfluß erhitzt. Man entfernt das Lösungsmittel am Rotationsverdampfer, versetzt mit Dimethylformamid und gießt auf Wasser. Es kristallisieren 2.85 g (73%) des 8-Brommethylchinolinderivats aus: Schmp.: 62—65°C.

c) 2.4 g 2,3-Bis(trifluormethyl-8-brommethyl-7-chlorchinolin suspendiert man in 6.8 g Propionsäure und 6.8 g 70%iger Schwefelsäure und tropft bei 120°C 5.5 g 65%ige Salpetersäure zu. Nach 2 Std. bei 120°C wird mit Wasser verdünnt, der Niederschlag wird abgesaugt, gewaschen un getrocknet; man isoliert 1,7 g(84%) 2,3-Bis(trifluormethyl)-7-chlorchinolin-8-carbonsäure; Schmp. 108—111°C.

Beispiele für erfindungsgemäße Verbindungen der Formel I sind in der folgenden Tabelle zusammengestellt:

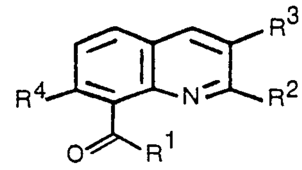

(I)

| Nr. | $R^1$ | $R^2$ | $R^3$ | $R^4$ | Fp [°C] |
|---|---|---|---|---|---|
| 1 | OH | H | $CF_3$ | Cl | 250 |
| 2 | $O-N=C(CH_3)_2$ | H | $CF_3$ | Cl | 105 - 107 |
| 3 | OH | $CF_3$ | $C_2H_5$ | Cl | 178 - 180 |
| 4 | OH | $CF_3$ | $CH_3$ | Cl | 186 - 188 |
| 5 | OH | $CF_3$ | $CF_3$ | Cl | 108 - 111 |
| 6 | $O-N=C(CH_3)_2$ | $CF_3$ | $CF_3$ | Cl | 91 |
| 7 | $O-N=C(C_2H_5)_2$ | H | $CF_3$ | Cl | |
| 8 | $O-N=C(CH_3)C_2H_5$ | H | $CF_3$ | Cl | |
| 9 | $O-N=C(C_3H_7)_2$ | H | $CF_3$ | Cl | |
| 10 | ONa | H | $CF_3$ | Cl | |
| 11 | OH | H | $CF_3$ | F | |
| 12 | OH | H | $CF_3$ | Br | |
| 13 | $OCH_3$ | H | $CF_3$ | Cl | |
| 14 | $OC_2H_5$ | H | $CF_3$ | Cl | |
| 15 | OH | H | $CHF_2$ | Cl | |
| 16 | OH | H | $CF_2-CH_3$ | Cl | |
| 17 | OH | H | $CH_2-CF_3$ | Cl | |
| 18 | OH | $CF_3$ | H | Cl | |
| 19 | OH | $CH_2-CF_3$ | H | Cl | |
| 20 | OH | $CH_3$ | $CF_3$ | Cl | |
| 22 | OH | H | $CH_2-CH_2-CF_3$ | Cl | |
| 23 | OH | $CF_3$ | $CH_3$ | Br | |
| 24 | OH | $CF_3$ | $C_2H_5$ | Br | 183 - 186 |
| 25 | OH | $CF_3$ | $CF_3$ | F | |

Die fluoralkylsubstituierten Chinolinderivate der Formel I können beispielsweise in Form von direkt verspürhbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können, die Substrate als solche oder in einem Öl oder Lösungsmittel gelöst, mittels Netz-, Haft-, Dispergier- oder Emulgiermittel in Wasser homogenisiert

werden. Es Können aber auch aus wirksamer Substanz Netz-, Haft-, Dispergier- oder Emulgiermittel und eventuell Lösungsmittel oder Öl bestehende Konzentrate hergestellt werde, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisazle, Salkze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes, Isoctylphenol, Octylphenol, oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produckte, wie Getreidemehl, Baumrinden-, Holz- und Nußschalenmehl, Cellulosepulver oder andere feste Trägerstoffe.

Die Formulierungen enthalten zwischen 0,1 und 95 Gew.% vorzugsweise zwischen 0,5 und 90 Gew.% Wirkstoff.

Beispiele für Formulierungen sind:

I. Man vermischt 90 Gewichtsteile der Verbindung Nr. 2 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 3 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 4 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 4 werden mit einer Mischung aus 92 Gewichtsteilen pulverförmigen Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. 40 Gewichtsteile des Wirkstoffs Nr. 2 werden in 60 Gewichtsteilen eine Mischung, die aus 93 Gew.% Xylol und 7 Gew.% des Anlagerungsproduktes von 8 Mol Ethylenoxid an 1 Mol Nonylphenol besteht, gelöst. Man erhält eine Lösung, die 40 Gew.% des Wirkstoffs enthält.

Die Applikation der Wirkstoffe bzw. der Mittel kann im Vorauflaufverfahren oder bei Nachauflaufanwendung erfolgen. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht

getroffen werden, während die Wirkstoffe aus die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium vorzugsweise 0,05 bis 5 kg/ha, vorzugsweise 0,05 bis 3 kg/ha.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 3% Humus als Substrat. Bei Soja werden größere Anteile Torf zugesetzt, um eine besseres Wachstum zu gewährleisten. Die Samen der Testpflanzen werden nach Arten getrennt flach eingesät. Unmittelbar danach werden bei Vorauflaufanwendung die Wirkstoffe auf die Erdoberfläche aufgebracht. Sie werden hierzu in Wasser als Verteilungsmittel suspendiert oder emulgiert und mittels fein verteilender Düsen gespritzt. Die Beispielsaufwandmengen betragen dabei 0,06, 0,125, 0,25 sowie 0,5 kg Wirkstoff/ha. Nach dem Aufbringen der Wirkstoffe werden die Gefäße leicht beregnet, um Keimung und Wachstum in Gang zu bringen. Danach werden die Gefäße mit durchsichtigen Plastikhauben abgedeckt, bis die Pflanzen angewachsen sind. Diese Abdeckung bewirkt eine gleichmäßiges Keimen der Testpflanzen, sofern diese nicht durch die Wirkstoffe beeinträchtigt werden.

Für die Nachauflaufbehandlung werden die Testpflanzen je nach Wuchsform erst bis zu einer Wuchshöhe von 3 bis 15 cm angezogen und danach behandelt. Zur Nachauflaufbehandlung werden entweder direkt gesäte und in den gleichen Gefäßen aufgewachsene Pflanzen ausgewählt, oder aber sie werden erst als Keimpflanzen in Saatschalen getrennt angezogen und einige Tage vor der Behandlung in die Versuchsgefäße verpflanzt. Eine Abdeckung unterbleibt bei der Nachauflaufbehandlung. Die Aufwandmengen für die Nachauflaufbehandlung betragen beispielsweise 3,0 und 0,5 kg Wirkstoff/ha.

Die Versuchsgefäße werden in Gewächshaus aufgestellt, wobei für wärmeliebende Arten wärmere Bereiche (20 bis 35°C) und für solche gmäßigter Klimate 10 bis 20°C bevorzugt werden. Die Versuchsperiode erstreckt sich bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

| | |
|---|---|
| Avena sativa | (Hafer) |
| Daucus carota | (Möhre) |
| Galium aparine | (Klettenlabkraut) |
| Glycine max | (Sojabohnen) |
| Ipomoea spp. | (Prunkwindearten) |
| Triticum aestivum | (Weizen) |
| Zea mays | (Mais). |

Als Vergleichsmittel dient die aus EP—A—O 104 389 bekannte Verbindung 3-Brommethyl-7-chlor-chinolin-8-carbonsäure (A).

Bei Vorauflaufanwendung ist die Verbindung Nr. 1 bei Karotten als Indikatorpflanzen wesentlich aktiver als das Vergleichsmittel A (Tab. 1). Karotten sind sehr sensible Bioindikatoren für herbizide Chinolincarbonsäuren und eignen sich zu deren biologischem Nachweis (Characteristics of the new Herbicides BAS 518 H, 1985 British Crop Protection Conference — Weeds 63—70. Außerdem bekämpft Verbindung Nr. 1 bei Vorauflaufanwendung unerwünschte breitblättrige Unkräuter, wie Ipomoea-Arten, selektiv in Soja (Tab. 2).

Bei Nachauflaufanwendung bekämpft die Verbindung Nr. 2 breitblättrige unerwünschte Pflanzen, ohne die Getreideart hafer zu schädigen (Tab. 3) Verbindung Nr. 1 ist bei Nachauflaufanwendung auch gegen unerwünschte breitblättrige Pflanzen wirksamer als das Vergleichsmittel A (Tab. 4); Weizen und Mais als Beispielskulturen werden dabei nicht geschädigt.

Die Testergebnisse sind den folgenden Tabellen zu entnehmen:

Tabelle 1

| Wirkstoff Nr. | Aufwandmenge [kg Wirkstoff/ha] | Schädigung [%] an Daucus carota |
|---|---|---|
| 1 | 0,25 | 100 |
| A | 0,25 | 45 |

Tabelle 2

| Wirkstoff Nr. | Aufwandmenge [kg Wirkstoff/ha] | Testpflanzen und Schädigung [%] Glycine max | Ipomoea spp. |
|---|---|---|---|
| 1 | 0,06 | 2 | 80 |
| | 0,125 | 6 | 89 |
| | 0,25 | 8 | 95 |
| | 0,5 | 11 | 96 |

Tabelle 3

| Wirkstoff Nr. | Aufwandmenge [kg Wirkstoff/ha] | Testpflanzen und Schädigung [%] Avena sativa | Galium aparine | Iopmoea spp. |
|---|---|---|---|---|
| 2 | 3,0 | 0 | 90 | 100 |

Tabelle 4

| Wirkstoff Nr. | Aufwandmenge [kg Wirkstoff/ha] | Testpflanzen und Schädigung [%] Triticum aestivum | Zea mays | Galium aparine | Ipomoea spp. |
|---|---|---|---|---|---|
| A | 0,5 | 0 | 10 | 60 | 20 |
| 1 | 0,5 | 0 | 0 | 90 | 80 |

In Anbetracht der guten Verträglichkeit für zahlreiche Kulturen und der Vielseitigkeit der Applikationsmethoden können die erfindungsgemäßen Chinolinderivate oder diese enthaltende Mittel noch in einer weiteren großen Zahl von Kulturpflanzen zur Beseitgung unerwünschten Pflanzenwuchses eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulg. | Zuckerrüben |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färbedistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Elaeis guineensis | Ölpalme |
| Fragaris vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |

| Botanischer Name | Deutscher Name |
|---|---|
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Sorghum bicolor (s. vulgare) | Mohrenhirse |
| Sorghum dochna | Zuckerhirse |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Zea mays | Mais |

Zur Verbreiterung des Wirkungsspektrums und zur Erzielung synergistischer Effekte können die Chinolinderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, andere Diphenylether, Triazinone, Uracile, Benzofuranderivate, Cyclohexan-1,3-dionderivate und andere herbizide Wirkstoffe in Betracht.

Außerdem ist es von Nutzen, die Chinolinderivate der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlöungen, welche zur Behebung von Ernährungs- und Spurenelementmängeln wie z.B. Ammoniumsulfat eingesetzt werden. Netzmittel (Surfactants) und nichtphytotoxische Öle und Ölkonzentrate können die herbizide Aktivität ebenfalls verbessern.

**Patentansprüche**

1. Fluoralkylsubstituierte Chinolinderivate der Formel

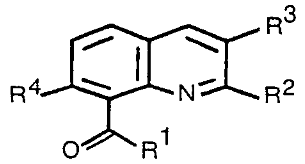

(I),

in der

$R^1$ für Chlor, Fluor, Hydroxy, $O^{\ominus}Met^{\oplus}$, $C_1$—$C_4$-Alkoxy oder einen

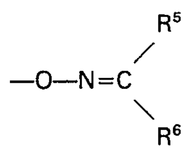

$R^2$, $R^3$ unabhängig voneinander für Wasserstoff, $C_1$—$C_3$-Alkyl, welches an einem der Kohlenstoffatome 2 oder Fluoratome tragen kann;

$R^4$ für Fluor, Chlor oder Brom stehen, wobei

$Met^{\oplus}$ ein Alkalimetallion und

$R^5$, $R^6$ unabhängig voneinander $C_1$—$C_3$-Alkyl bedeuten,

mit der Maßgabe, daß mindestens einer der Reste $R^2$ und $R^3$ $C_1$—$C_3$-Fluoralkyl bedeutet.

2. 3-Trifluormethyl-7-chlorchinolin-8-carbonsäure.

3. Verfahren zur Herstellung von fluoralkylsubstituierten Chinolinderivaten der Formel I gemäß Anspruch 1, in denen $R^1$ Hydroxy bedeutet, dadurch gekennzeichnet, daß man Chinolinderivate der Formel II

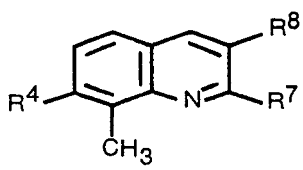

(II),

in der

$R^4$ die im Anspruch 1 genannten Bedeutungen hat, und mindestens einer der Substituenten

$R^7$ oder $R^8$ für Formyl, Acetyl, Carboxyl, Carboxymethyl, Carboxyethyl oder Formylmethyl steht und gegebenenfalls der andere Substituent Wasserstoff oder $C_1$—$C_3$-Alkyl bedeutet, mit Schwefeltetrafluorid umsetzt und die Verbindungen anschließend in an sich bekannter Weise oxidiert.

4. Verfahren zur Herstellung von fluoralkylsubstituierten Chinolinderivate der Formel I gemäß 1 Anspruch 1, wobei $R^1$ Hydroxyl, $R^2$ Trifluormethyl und $R^3$ $C_1$—$C_3$-Alkyl bedeuten, dadurch gekennzeichnet, daß man 3,1-Benzoxazin-4-one der Formel III

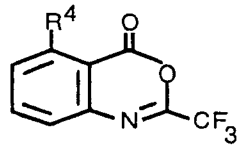

(III),

in der

$R^4$ die im Anspruch 1 genannten Bedeutungen hat, mit Enaminen der Formel IV

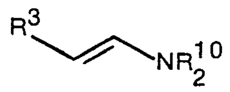

(IV),

in der

$R^3$ $C_1$—$C_3$-Alkyl und $NR_2^{10}$ Di-$C_1$—$C_3$-alkylamino bedeutet, in an sich bekannter Weise umsetzt.

5. Herbizid, enthaltend ein fluoralkylsubstituiertes Chinolinderivat der Formel I gemäß Anspruch 1.

6. Herbizid, enthaltend inerte Zusatzstoffe und eine fluoralkylsubstituiertes Chinolinderivat der Formel I gemäß Anspruch 1.

7. Verfahren zur Bekämpfung unerwünschten Pflanzenwuchses, dadurch gekennzeichnet, daß man die unerwünschten Pflanzen oder die von unerwünschtem Pflanzenwuchs freizuhaltende Fläche mit einer herbizid wirksamen Menge eines fluoralkylsubstituierten Chinolinderivats der Formel I gemäß Anspruch 1 behandelt.

**Revendications**

1. Dérivés de quinoléine à substituants fluoroalkyle de formule

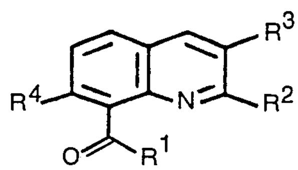

(I),

dans laquelle

$R^1$ est mis pour chlore, fluor, hydroxy, $O^{\ominus}Met^{\oplus}$, alcoxy en $C_1$—$C_4$ ou un

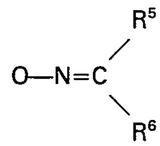

$R^2$, $R^3$ indépendamment l'un de l'autre, pour hydrogène, alkyle en $C_1$—$C_3$, qui peut porter 2 ou 3 atomes de fluor sur des atomes de carbone;

$R^4$ pour fluor, chlore ou brome,

$Met^{\oplus}$ représentant un ion de métal alcalin et

$R^5$, $R^6$ indépendamment l'un de l'autre, alkyle en $C_1$—$C_3$, avec la condition qu'au moins un des restes $R^2$ et $R^3$ signifie fluoro-alkyle en $C_1$—$C_3$.

2. Acide 3-trifluorométhyl-7-chloroquinoléine-8-carboxylique.

3. Procédé de préparation de dérivés de quinoléine à substituants fluoroalkyle de formule I selon la revendication 1 dans laquelle $R^1$ signifie hydroxy caractérisé par le fait qu'on fait réagir avec du tétrafluorure de soufre, des dérivés de quinoléine de formule II

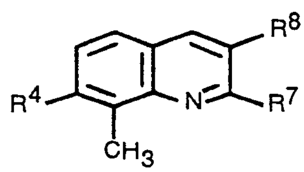

(II),

dans laquelle

$R^4$ a les significations indiquées dans la revendication 1, et au moins un des substituants $R^7$ ou $R^8$ est mis pour formyle, acétyle, carbonyle, carboxyméthyle, carboxyéthyle ou formylméthyle et, éventuellement, l'autre substituant signifie hydrogène ou alkyle en $C_1$—$C_3$, et l'on oxyde ensuite les composés d'une manière connue en soi.

4. Procédé de préparation de dérivés de quinoléine à substituants fluoroalkyle de formule I selon la revendication 1, $R^1$ représentant hydroxyle, $R^2$, trifluorométhyle et $R^3$ alkyle en $C_1$—$C_3$, caractérisé par le fait qu'on fait réagir, de manière connue en soi, de la 3,1-benzoxazin-4-one de formule III

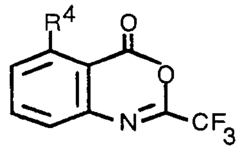

(III),

dans laquelle

$R^4$ a les significations indiquées dans la revendication 1 avec des énamines de formule IV

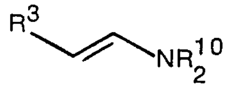

(IV),

dans laquelle

$R^3$ signifie alkyle en $C_1$—$C_3$ et $NR_2^{10}$, di-(alkyl en $C_1$—$C_3$)-amino.

5. Herbicide, contenant un dérivé de quinoléine à substituants fluoroalkyle de formule 1 selon la revendication 1.

6. Herbicide contenant des additifs inertes et un dérivé de quinoléine à substituants fluoroalkyle de formule I selon la revendication 1.

7. Procédé de lutte contre la croissance de plantes indésirables, caractérisé par le fait qu'on traite les plantes indésirables ou les surfaces à maintenir libres de la croissance de plantes indésirables avec une quantité efiicace du point de vue herbicide d'un dérivé de quinoléine à substituants fluoroalkyle de formule I selon la revendication 1.

**Claims**

1. A fluoroalkyl-substituted quinoline derivative of the formula

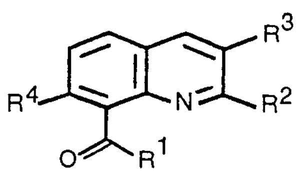

(I),

where $R^1$ is chlorine, fluorine, hydroxyl, $O^{\ominus}Met^{\oplus}$, $C_1$—$C_4$-alkoxy or a radical of the formula

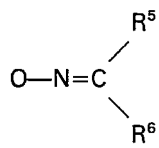

$R^2$ and $R^3$ independently of one another are each hydrogen or $C_1$—$C_3$-alkyl which can carry 2 or 3 fluorine atoms on one of the carbon atoms;

$R^4$ is fluorine, chlorine or bromine,

$Met^{\oplus}$ is an alkali metal ion and

$R^5$ and $R^6$ independently of one another are each $C_1$—$C_3$-alkyl, with the proviso that one or both of the radicals $R^2$ and $R^3$ are $C_1$—$C_3$-fluoroalkyl.

2. 3-Trifluoromethyl-7-chloroquinoline-8-carboxylic acid.

3. A process for the preparation of fluoralkylsubstituted quinoline derivatives of the formula I as claimed in claim 1, in which $R^1$ is hydroxyl, wherein a quinoline derivative of the formula II

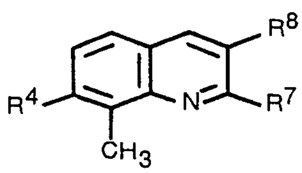

(II),

where $R^4$ has the meanings given in claim 1, and at least one of the substituents

$R^7$ or $R^8$ is formyl, acetyl, carboxyl, carboxymethyl, carboxyethyl or formylmethyl, and the other substituent may be hydrogen or $C_1$—$C_3$-alkyl, is reacted with sulfur tetrafluoride, and the compounds are subsequently oxidized in a conventional manner.

4. A process for the preparation of fluoroalkylsubstituted quinoline derivatives of the formula I as claimed in claim 1, $R^1$ denoting hydroxyl, $R^2$ denoting trifluoromethyl and $R^3$ denoting $C_1$—$C_3$-alkyl, wherein a 3,1-benzoxazin-4-one of the formula III

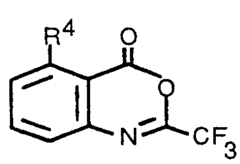

(III),

where $R^4$ has the meanings given in claim 1, is reacted in a conventional manner with an enamine of the formula IV

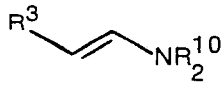

(IV),

where $R^3$ is $C_1$—$C_3$-alkyl and $NR_2^{10}$ is a di-$C_1$—$C_3$-alkylamino.

5. A herbicide containing a fluoroalkyl-substituted quinoline derivative of the formula I as claimed in claim 1.

6. A herbicide containing inert additives and a fluoroalkyl-substituted quinoline derivative of the formula I as claimed in claim 1.

7. A method for controlling undesirable plant growth, wherein the undesirable plants or the area to be kept free from undesirable plant growth are or is treated with a herbicidally effective amount of a fluoroalkylsubstituted quinoline derivative of the formula I as claimed in claim 1.